# FASCICULE DE BREVET EUROPEEN

(11) **EP 1 111 389 B1**
(45) Date de publication et mention de la délivrance du brevet: **01.03.2006**
(21) Numéro de dépôt: 00403310.6
(22) Date de dépôt: 04.12.2000
(51) Int. Cl.: G01N 33/68, G01N 33/53

(54) **Utilisation d'un anticorps spécifique des fibroblastes papillaires comme marqueur de qualité d'une peau**
Verwendung eines Papillarfibroblast-spezifischen Antikörpers als Marker der Hautqualität
Use of an antibody specific for papillary fibroblasts as a marker for skin quality

(30) Priorité: 03.12.1999 FR 9915292
(43) Date de publication de la demande: 27.06.2001
(73) Titulaire: L'OREAL, 75008 Paris (FR)
(72) Inventeur: Asselineau, Daniel, 92160 Antony (FR); Caplan, Arnold, Cleveland, Ohio 44106 (US)
(74) Mandataire: Allab, Myriam

(56) Documents cités:
- SORRELL MICHAEL J; CARRINO DAVID A; BABER MARILYN A; ASSELINEAU DANIEL; CAPLAN ARNOLD I: "A monoclonal antibody which recognizes a glycosaminoglycan epitope in both dermatan sulfate and chondroitin sulfate proteoglycans of human skin" HISTOCHEMICAL JOURNAL, vol. 31, août 1999 (1999-08), pages 549-558, XP000938231
- SORRELL J M; MAHMOODIAN F; SCHAFER I A; DAVIS B; CATERSON B: "IDENTIFICATION OF MONOCLONAL ANTIBODIES THAT RECOGNIZE NOVEL EPITOPES IN NATIVE CHONDROITIN-DERMATAN SULFATE GLYCOSAMINOGLYCAN CHAINS THEIR USE IN MAPPING FUNCTIONALLY DISTINCT DOMAINS OF HUMAN SKIN" JOURNAL OF HISTOCHEMISTRY AND CYTOCHEMISTRY, vol. 38, 1990, pages 393-402, XP000937833
- SCHONHERR E ET AL: "Differences in decorin expression by papillary and reticular fibroblasts in vivo and in vitro." BIOCHEMICAL JOURNAL, (1993 MAR 15) 290 ( PT 3) 893-9., XP000938060
- HUNZELMANN NICOLAS; SCHOENHERR ELISABETH; BONNEKOH BERND; HARTMANN CAROLINE; KRESSE HANS; KRIEG THOMAS: "Altered Immunohistochemical Expression of Small Proteoglycans in the Tumor Tissue and Stroma of Basal Cell Carcinoma" JOURNAL OF INVESTIGATIVE DERMATOLOGY, vol. 104, 1995, pages 509-513, XP000937837
- WILLEN M D; SORRELL J M; LEKAN C C; DAVIS B R; CAPLAN A I: "PATTERNS OF GLYCOSAMINOGLYCAN PROTEOGLYCAN IMMUNOSTAINING IN HUMAN SKIN DURING AGING" JOURNAL OF INVESTIGATIVE DERMATOLOGY, vol. 96, 1991, pages 968-974, XP000937841
- HACHISUKA HIROSHI; YAMAMOTO NOBUHIRO; SAKIHAMA HIDEKI; SASAI YOICHIRO: "Proteoglycans in albo-papuloid lesions of the Pasini form of dominant dystrophic epidermolysis bullosa" KURUME MEDICAL JOURNAL, vol. 42, 1995, pages 1-8, XP000937849
- ASSELINEAU D; TECHER M; CAPLAN A; SORRELL J; DEMARCHEZ M; JOMARD A; ROSSIO P; REINICHE P: "Complex reconstructed skin equivalents made with papillary and reticular fibroblast populations incorporated in distinct layers: Re-expression of papillary and reticular fibroblast characteristics after grafting onto nude mice" JOURNAL OF INVESTIGATIVE DERMATOLOGY, vol. 114, avril 2000 (2000-04), page 863 XP000937959
- ASSELINEAU D; BERNARD B A; BAILLY C; DARMON M; PRUNIERAS M: "HUMAN EPIDERMIS RECONSTRUCTED BY CULTURE IS IT NORMAL" JOURNAL OF INVESTIGATIVE DERMATOLOGY, vol. 86, 1986, pages 181-186, XP000937840

## Description

L'invention a pour objet l'utilisation d'au moins un anticorps spécifique des fibroblastes papillaires comme marqueur de qualité d'une peau, particulièrement d'un équivalent de peau.

La peau est constituée de deux compartiments intimement liés, à savoir l'épiderme et le derme.

L'épiderme est majoritairement constitué de trois types cellulaires, les kératinocytes eux-mêmes majoritaires parmi les cellules de l'épiderme, les mélanocytes et les cellules de Langerhans. Ces cellules constituent un épithélium kératinisé qui se différencie en couches superposées surmontées d'une couche de cellules mortes formant le stratum corneum.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale. L'ensemble de ces composants extracellulaires est synthétisé par le fibroblaste. On y trouve aussi des leucocytes, des mastocytes ou encore des macrophages tissulaires. Il est également constitué de vaisseaux sanguins et de fibres nerveuses. Dans une peau normale, c'est à dire ni pathologique ni cicatricielle, le fibroblaste est à l'état quiescent, c'est à dire non prolifératif, peu actif d'un point de vue métabolique et non mobile.
En fait le derme se subdivise en deux régions, d'une part un derme superficiel fin appelé papillaire, d'autre part le derme profond appelé réticulaire qui constitue la grande majorité du derme.
Le derme papillaire est la partie du derme située au contact de l'épiderme et il contient des fibroblastes dit papillaires.
Le derme réticulaire est la région du derme s'étendant ensuite jusqu'à la couche graisseuse sous-cutanée et il contient des fibroblastes dits réticulaires. Ces deux régions présentent dans la peau normale des différences. Le derme papillaire est métaboliquement plus actif que la derme réticulaire.
Les fibroblastes papillaires et réticulaires en culture présentent des différences dans leur potentiel de croissance. Par immunomarquage il est possible de montrer que la décorine, dermatane sulfate protéoglycan (DSPG) de petite taille, est plus abondante dans le derme papillaire que dans le derme réticulaire. Les fibroblastes papillaires sécrètent jusqu'à environ 6 fois plus de décorine que les fibroblastes réticulaires.

Donc dans la peau normale le derme est constitué d'au moins deux populations de fibroblastes ce qui ne peut qu'avoir des conséquences essentielles sur la peau elle-même.

Dans le domaine des équivalents de peaux (ou peaux reconstruites in vitro), on sait préparer des équivalents de derme avec chacune des populations de fibroblastes préalablement isolées. On sait également préparer des équivalents de derme dans lesquels les deux populations préalablement isolées sont introduites.
Mais le problème demeure de l'identification des différentes populations de fibroblastes dans des équivalents de derme reconstruits à partir d'une population aléatoires de fibroblastes. Après établissement en culture de l'équivalent de derme, ce dernier présente-t-il au moins les deux populations de fibroblastes, réticulaire et papillaire, présents dans le derme d'une peau normale ? Or on a vu précédemment que la peau normale présente ces deux populations et l'on comprend qu'une peau reconstruite in vitro sera d'autant plus proche d'une peau normale quand elle présentera au moins les deux populations de fibroblastes.

A ce jour à la connaissance de la demanderesse il n'existe pas de moyen simple et efficace, particulièrement de moyen non invalidant pour l'équivalent de peau qui permettent d'établir sans ambiguïté si une peau reconstruite présente ou non au moins les deux populations de fibroblastes papillaire et réticulaire. C'est ce problème que vise à résoudre la présente invention.

En effet de manière surprenante et inattendue la demanderesse a maintenant découvert que les fibroblastes papillaires exprime un épitope spécifique qui n'est pas, ou peu, présent chez les fibroblastes réticulaires. Il est alors possible d'effectuer à l'aide d'anticorps, particulièrement d'anticorps monoclonaux, spécifiques de cet épitope un marquage de cette population particulière de fibroblastes dermiques. Ainsi, il est possible à l'aide de cet anticorps de déterminer si un équivalent de peau présente les deux populations de fibroblastes papillaire et réticulaire.

Ainsi, l'invention a pour objet l'utilisation d'au moins un anticorps spécifique des fibroblastes papillaires comme marqueur de qualité d'une peau, particulièrement d'un équivalent de peau.

Par marqueur de qualité on entend selon l'invention, tout marqueur qui indique effectivement la présence dans la peau ou dans l'équivalent de peau, d'un élément biologique présent dans la peau normale.

Par marqueur on entend selon l'invention, tout élément dont la présence, l'absence, la modification de l'expression ou la modification de la distribution peuvent être mesurées. On peut citer à titre d'exemple de marqueur, et sans limitation, les épitopes, les acides nucléiques (acide ribonucléique ou désoxyribonucléïque), les anticorps, les protéines ou groupe de protéines liées ou non, les ions, les organites cellulaires, les lipides ou les polyosides.
Selon l'invention le marqueur est un anticorps.

L'anticorps peut être un anticorps polyclonal ou monoclonal. Préférentiellement selon l'invention l'anticorps est monoclonal.

L'anticorps peut être un anticorps provenant de toute origine c'est à dire issu de n'importe quel animal comme par exemple le cheval, la chèvre, la souris, le rat, le lapin.
Préférentiellement l'anticorps est un anticorps de souris.
Encore plus préférentiellement l'anticorps est un anticorps monoclonal de souris.

Un anticorps préféré selon l'invention est l'anticorps connu sous le nom de PG4, décrit dans la publication de Sorrell et col. (The Histochemical Journal 31 : 549-558, 1999).
Cet anticorps monoclonal de souris est décrit comme reconnaissant au niveau de la peau au moins un épitope spécifique des glycosaminoglycans, particulièrement décrit comme un anticorps monoclonal anti-chondroitine sulfate (CS) et antidermatane sulfate (DS).
A ce jour cet anticorps n'a pas été décrit comme spécifique d'une population particulière de fibroblates dermiques à savoir les fibroblastes papillaires.
Sorrell et al (Journal of Histochemistry and Cytachemistry, 1990, 393-402) décrit des anticorps monoclonaux qui reconnaissent des épitopes de chondroitine ou dermatane sulfate et leur utilisation pour le marquage de la peau.
Schönherr et al (Biochem. 1993, **290,** 893-899) rapporte des différences d'expression de certains protéoglycanes dans les fibroblastes papillaires et réticulaires.
Hunzelman et al (J. Invest. Dermatol., 1995, **104,** 509-513) décrivent l'expression altérée de protéoglycanes dans des tissus tumoraux.

Ainsi l'invention a pour objet l'utilisation de l'anticorps monoclonal PG4 comme marqueur des fibroblastes papillaires, particulièrement des fibroblastes papillaires de la peau, très particulièrement fibroblastes papillaires du derme.

Un autre objet de l'invention est l'utilisation de l'anticorps monoclonal PG4 comme marqueur de qualité de la peau, particulièrement des équivalents de peau, particulièrement des équivalent de derme obtenus in vitro.

Toute méthode de marquage immunologique utilisant au moins un anticorps, connue de l'art antérieur peut être utilisée pour réaliser les marquages avec les anticorps de l'invention. On peut à cet égard citer la méthode décrite par Asselineau et col., (J. I. D., 86, 181-186, 1986), ou encore par Sorrell et col. (The Histochemical Journal 31 : 549-558, 1999).

Un autre objet de l'invention est un procédé de détermination de la qualité d'une peau, particulièrement d'un équivalent de peau obtenu in vitro, caractérisé en ce que l'on effectue sur la peau et/ou l'équivalent de peau un marquage immunologique à l'aide d'au moins un anticorps spécifique des fibroblastes papillaires, particulièrement de l'anticorps PG4.

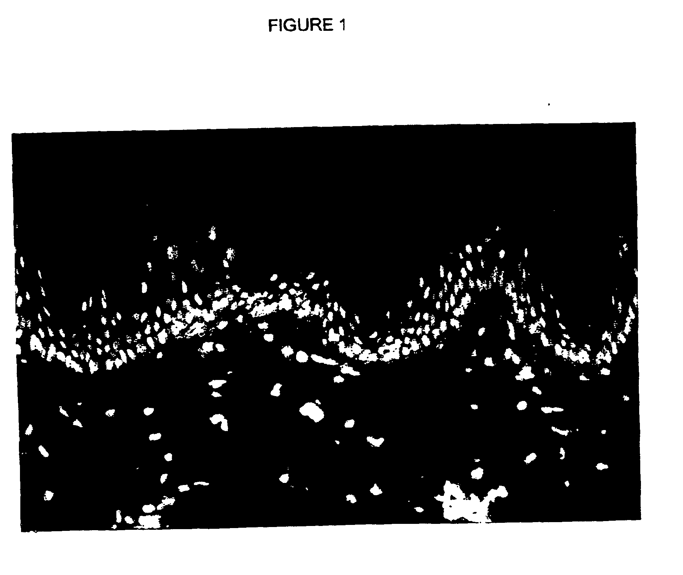

La figure 1 permet de mieux illustrer l'invention, sans toutefois en limiter sa portée.
Dans cette figure, la photo montrent un coupe de peau humaine normale après immunomarquage réalisé en immunofluorescence indirecte à l'aide de l'anticorps monoclonal PG4 avec contremarquage des noyaux cellulaires au iodure de propidium. On note la présence d'un marquage intense (zone grisée claire) du derme supérieur au niveau de l'épiderme (reconnaissable au nombreux noyaux cellulaires marqués au iodure de propidium), démontrant la présence dans cette zone de fibroblastes papillaires exprimant l'épitope reconnu spécifiquement par l'anticorps monoclonal PG4.

D'autres caractéristiques et avantages de l'invention ressortiront mieux des exemples qui suivent, donnés à titre illustratif et non limitatif.

### Exemple 1 : Marquage immunologique des fibroblastes papillaires dans une peau normale.

Des échantillons de peau normale issus de plasties chirurgicales sont inclus dans le Tissue-Tek, congelés en azote liquide et conservés au congélateur à -80°C.
Des coupes de 4 microns d'épaisseur sont réalisées au cryostat selon les techniques standards.
Les marquages sont réalisés par une technique classique de marquage en immunofluorescence indirecte (voir Asselineau et col., J. I. D., 86, 181-186, 1986) avec 20µl par coupe de l'anticorps monoclonal PG4 utilisé pur (surnageant de culture) (voir Sorrell et col. The Histochemical Journal 31 : 549-558, 1999). 20 µl par coupe d'un anticorps dirigé contre les anticorps de souris (conjugué de souris) obtenu auprès de la société Dako sont alors déposés sur les coupes et celles-ci sont incubées selon les recommandations du fournisseur.
Après rinçage, les coupes sont soumises à une solution de PBS contenant du iodure de propidium à 0,5% puis rincées au PBS et montées pour observation au microscope à fluorescence afin de marquer les noyaux cellulaires.

On note la présence d'un marquage intense du derme au niveau de l'épiderme, démontrant la présence dans cette zone de fibroblastes papillaires exprimant l'épitope reconnu spécifiquement par l'anticorps monoclonal PG4.

## Revendications

1. Utilisation de l'anticorps PG4, ledit anticorps étant spécifique des fibroblastes papillaires, comme marqueur de qualité d'une peau pour indiquer la présence dans la peau de populations de fibroblastes de peau normale.

2. Utilisation de l'anticorps PG4, ledit d'anticorps étant spécifique des fibroblastes papillaires, comme marqueur de qualité d'un équivalent de peau pour indiquer la présence dans l'équivalent de peau de populations de fibroblastes de peau normale.

3. Utilisation selon l'une quelconque des revendications 1 ou 2, **caractérisée par le fait que** l'anticorps est un anticorps monoclonal.

4. Utilisation de l'anticorps PG4 comme marqueur des fibroblastes papillaires.

5. Utilisation selon la revendication 4, **caractérisée par le fait que** les fibroblastes sont des fibroblastes papillaires de peau.

6. Utilisation selon l'une quelconque des revendications 4 ou 5, **caractérisée par le fait que** les fibroblastes sont des fibroblastes papillaires du derme.

7. Utilisation de l'anticorps PG4 selon la revendication 1, comme marqueur de qualité d'un derme ou d'un équivalent de derme.

8. Procédé de détermination de la qualité d'une peau ou d'un équivalent de peau obtenu in vitro, **caractérisé en ce que** l'on effectue sur cette peau ou sur cet équivalent de peau un marquage immunologique à l'aide de l'anticorps PG4, comme anticorps spécifique des fibroblastes papillaires.

## Patentansprüche

1. Verwendung des Antikörpers PG4, wobei der Antikörper spezifisch für Papillarfibroblasten ist, als Marker für die Hautqualität, um die Gegenwart von Fibroblastenpopulationen der normalen Haut in der Haut anzuzeigen.

2. Verwendung des Antikörpers PG4, wobei der Antikörper spezifisch für Papillarfibroblasten ist, als Marker für die Qualität eine Hautäquivalents, um die Gegenwart von Fibroblastenpopulationen normaler Haut in dem Hautäquivalent anzuzeigen.

3. Verwendung nach einem der Ansprüche 1 oder 2, **dadurch gekennzeichnet, dass** der Antikörper ein monoklonaler Antikörper ist.

4. Verwendung des Antikörpers PG4 als Marker für Papillarfibroblasten.

5. Verwendung nach Anspruch 4, **dadurch gekennzeichnet, dass** die Fibroblasten Papillarfibroblasten der Haut sind.

6. Verwendung nach einem der Ansprüche 4 oder 5, **dadurch gekennzeichnet, dass** die Fibroblasten Papillarfibroblasten der Dermis sind.

7. Verwendung des Antikörpers PG4 nach Anspruch 1 als Marker für die Qualität einer Dermis oder eines Dermisäquivalents.

8. Verfahren zur Bestimmung der Hautqualität oder der Qualität eines *in vitro* hergestellten Hautäquivalents, **dadurch gekennzeichnet, dass** an der Haut oder dem Hautäquivalent eine immunologische Markierung mit Hilfe des Antikörpers PG4 als für Papillarfibroblasten spezifischer Antikörper durchgeführt wird.

## Claims

1. Use of the PG4 antibody, the said antibody being specific for papillary fibroblasts, as a marker for the quality of skin, for indicating the presence in the skin of fibroblast populations of normal skin.

2. Use of the PG4 antibody, the said antibody being specific for papillary fibroblasts, as a marker for the quality of a skin equivalent, for indicating the presence in the skin equivalent of fibroblast populations of normal skin.

3. Use according to either of Claims 1 and 2, **characterized in that** the antibody is a monoclonal antibody.

4. Use of the PG4 antibody as a marker for papillary fibroblasts.

5. Use according to Claim 4, **characterized in that** the fibroblasts are papillary fibroblasts of skin.

6. Use according to either of Claims 4 and 5, **characterized in that** the fibroblasts are papillary fibroblasts of the dermis.

7. Use of the PG4 antibody according to Claim 1, as a marker for the quality of a dermis or of a dermis equivalent.

8. Method for determining the quality of skin or of a skin equivalent obtained in vitro, **characterized in that** immunological labelling is performed on this skin or on this skin equivalent using the PG4 antibody, as antibody specific for papillary fibroblasts.
